(19) Europäisches Patentamt
European Patent Office
Office européen des brevets

(11) **EP 3 153 981 A1**

(12) **EUROPEAN PATENT APPLICATION**

(43) Date of publication:
**12.04.2017 Bulletin 2017/15**

(51) Int Cl.:
***G06F 19/00*** *(2011.01)*

(21) Application number: **15306560.2**

(22) Date of filing: **05.10.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicant: **Movea**
**38000 Grenoble (FR)**

(72) Inventors:
• **RICCARDI, Sébastien**
  **38590 BREZINS (FR)**
• **CUBIZOLLE, Thomas**
  **38000 GRENOBLE (FR)**
• **POIRIER, Grégory**
  **73190 CHALLES-LES-EAUX (FR)**

(74) Representative: **Brunelli, Gérald**
**Marks & Clerk France**
**Immeuble Visium**
**22, avenue Aristide Briand**
**94117 Arcueil Cedex (FR)**

(54) **METHOD AND SYSTEM FOR ESTIMATING A POSITION OF A BALL IMPACT ON A RACKET STRING BED**

(57) Method for estimating a position of a ball impact on a racket string bed, along the longitudinal axis (Y) of the racket (1), from data provided by a proximity sensor unit. The proximity sensor unit may comprise an acceleration sensor or an optical sensor to determine the ball impact. In addition, the ball impact along the transversal axis (X) of the racket (1) may be determined based on gyrometer data.

FIG.1

EP 3 153 981 A1

**Description**

The invention relates to the field of analysis of a ball impact on a racket string bed.

Electronic tennis rackets exist where accelerometers and gyrometers are incorporated in the handle in order to analyze the tennis swings of the user. In addition, piezo sensors embedded in the racket handle enable to determine the impact of the ball on the string bed. The patent application US US2015/0157901 A1 discloses many elements of such an electronic racket.

The use of a piezo sensor embedded in the racket handle to determine a position of a ball impact on a racket string bed has a high cost because the sensor needs to be integrated in the racket handle.

Thus such a racket is more expensive than a conventional racket, and a player has to buy a new racket if he wants to use such an innovation.

An aim of embodiments of the present invention is to propose a method and a system with a reduced cost, adaptable to any racket. In an embodiment of the invention, the system is an add-on module that can be added by the player to any racket.

It is proposed according to an aspect of the invention, a method for estimating a position of a ball impact on a racket string bed, along the longitudinal axis of the racket, from data provided by a proximity sensor unit.

Such a method enables to determine the impact position by calculating the distance between the proximity sensor unit and the ball during the impact.

According to an embodiment, said data are provided by an accelerometer.

The use of an accelerometer enables a very power efficient method of determining the impact position in a compact system. In addition, the accelerometer may be used in the calculation of the orientation of the racket, which means that the accelerometer is used for a multitude of functionalities.

According to an embodiment, said position of a ball impact on the racket string bed, along the longitudinal axis of the racket, is estimated using a frequency analysis of data provided by the accelerometer.

Such an embodiment provides a way to obtain information about the impact position based on an analysis of the Fast Fourier Transform (FFT) of the accelerometer signal.

According to an embodiment, if the frequency of the first minimum magnitude in the FFT curve corresponding to the provided data is greater than the frequency of the first maximum magnitude of the FFT curve corresponding to the provided data, then the position of the ball impact is estimated more at the top of the racket string bed, else the position of the ball impact is estimated more at the yoke of the racket string bed.

Such an embodiment enable to use the position of the maximum with respect to the minimum to determine whether the impact position is in the top or bottom part of the racket.

According to an embodiment, if the position of the ball impact is estimated more at the top of the racket string bed, and if the difference between said first maximum magnitude and the magnitude at a fixed frequency is greater than a first threshold, then the position of the ball impact is estimated at the top of the racket string bed, else the position of the ball impact is estimated at the center of the racket string bed.

For instance, the fixed frequency can be 25 Hz.

Such an embodiment enables to use the magnitude of the maximum with respect to the magnitude at a fixed frequency to determine whether the impact position is in the top or center part of the racket.

According to an embodiment, if the position of the ball impact is estimated more at the yoke of the racket string bed and if the difference between said first maximum magnitude and said first minimum magnitude is greater than a second threshold, then the position of the ball impact is estimated at the yoke of the racket string bed, else the position of the ball impact is estimated at the center of the racket string bed.

Such embodiments use the magnitudes of the peaks in addition to the frequency positions in order to determine the impact position with a greater accuracy than based on the frequency positions of the peaks alone.

According to an embodiment, said data are provided by an optical sensor comprising at least one light source and at least one light detector.

The use of such an optical sensor enables to determine the impact position independent of any mechanical or vibrational characteristics of the racket frame or string bed.

According to an embodiment, said position along the longitudinal axis of the racket is determined from the time of flight of the signals of the optical sensor. The time of flight method is a standard method to determine a distance between the detector and an object by calculating the time it takes the light emitted by the detector, to reflect off the object, and be detected by the sensor.

Such an embodiment makes the system independent of any variations of the light intensity of the reflections, or any ambient light.

According to an embodiment, said position along the longitudinal axis of the racket is determined from analyzing the amplitude of the signals of the optical sensor.

For example, the reflection of the light emitted from the light source by the ball as measured by the optical sensor is used to determine the impact position.

Such an embodiment needs less critical time components than when a time of flight method is used.

According to an embodiment, a calibration is effected to match the amplitude of the reflection and the corresponding position of a ball impact on the racket string bed, along the longitudinal axis of the racket.

Such an embodiment may allow for a calibration of the position of the sensor with respect to the string bed.

According to an embodiment, a measurement is effected with the light source off.

Such an embodiment allows to measure the amount of ambient light and to correct its influence.

According to an embodiment, a measurement is effected in absence of a ball impact.

Such an embodiment allows to correct for any possible reflections from the racket frame and string bed.

According to an embodiment, the light source is on, only during a racket swing detected from data provided by a gyrometer.

Such an embodiment powers the light source only when an impact is expected in order to save battery power.

According to an embodiment, the gyrometer measures the angular velocity for a rotation along the longitudinal axis of the racket.

Such an embodiment enables to determine the lateral impact position of the ball.

According to an embodiment, the light source is controlled based on data provided by a gyrometer or an accelerometer.

Such an embodiment enables to optimize the battery power by only powering the light source for a narrow window before and/or after the impact.

According to an embodiment, the light source is controlled based on data provided by at least one of a gyrometer and an accelerometer.

According to an embodiment, a type of swing is determined from data provided by at least one of a gyrometer and an accelerometer.

Such an embodiment may be used to give feedback to the user on the impact position for different types of swing.

According to an embodiment, the light source is controlled based on type of swing detected.

Such an embodiment may enable to power only the required light source in case there is a plurality of light sources.

According to an embodiment, the light source is on, during an interval around a detected ball impact on a racket string bed.

Such an embodiment enables to limit power used by the light source.

According to an embodiment, at least one of an incoming ball speed and an outgoing ball speed is determined based on said data.

According to an embodiment, the method comprises an estimation of a transversal position of a ball impact among a transversal axis (X), for example defined as a left, a center, and a right positions.

It is also proposed, according to another aspect of the invention, a system for estimating a position of a ball impact on a racket string bed, along the longitudinal axis of the racket, comprising :

- an add-on module adapted to be mounted on a racket, the add-on module comprises a proximity sensor unit;
- a calculator adapted to estimate the position of a ball impact on the racket string bed, along the longitudinal axis of the racket, from data provided by the proximity sensor unit.

According to an embodiment, the proximity sensor comprises an accelerometer.

According to an embodiment, the proximity sensor comprises at least one light source and at least one light detector.

According to an embodiment, the proximity sensor comprises two light sources configured to be disposed on each side of the racket.

According to an embodiment, the system comprises a gyrometer.

The invention will be better understood with the discussion of some embodiments described by way of non-limiting examples and illustrated by the accompanying drawings wherein :

- figure 1 illustrates an example of a tennis racket, with possible positions for an add-on module of a system according to an aspect of the invention ;
- figure 2a illustrates a separation of the impact position in three sections (left, center and right) of the racket string bed, according to a transversal axis X;
- figure 2b represents the variation of the angular rotational velocity as measure by a gyrometer along the longitudinal axis Y during an impact of the projectile, according to an aspect of the invention;
- figures 3 to 6 illustrate data provided by an accelerometer;
- figure 7 illustrates a bounce of a ball within a light beam, according to an aspect of the invention;
- figure 8 illustrates a method of analyzing the amplitude of the signals of the optical sensor, according to an aspect of the invention; and
- figure 9 illustrates a system according to an aspect of the invention.

The following figures explain in more detail the functioning of the present invention.

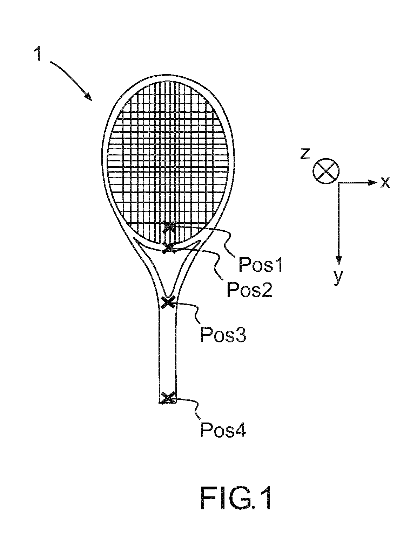

Figure 1 shows a tennis racket 1, wherein four example positions Pos1, Pos2, Pos3, and Pos4 are represented where an add-on module of a system according to the invention may be positioned. A first position Pos 1 is at the bottom of the string bed, close to the yoke of the racket, a second position Pos2 is at the yoke part of the racket, a third position Pos3 is just above the top of the handle of the tennis racket, and a fourth position Pos4 is at the bottom of the racket handle.

A reference frame is associated with the racket, defining three orthogonal axis, an X axis, an Y axis, and a Z axis.

The Y axis represents a longitudinal axis of the racket, the X axis represent a transversal axis, orthogonal to the longitudinal axis Y, the plane defined by the X and Y axis corresponds to the string bed of the racket. The Z axis is an axis orthogonal to the string bed of the racket and forming a direction oriented orthogonal with reference to the X axis and Y axis.

The system according to an aspect of the invention allows to estimate a position of a ball impact on a racket string bed, comprising :

- an add-on module adapted to be mounted on a racket, the add-on module comprises a proximity sensor unit;
- a calculator adapted to estimate the position of a ball impact on the racket string bed, from data provided by the proximity sensor unit.

The impact position may be determined along the longitudinal axis (Y axis) and along the transversal axis (X axis). The proximity sensor unit may contain different sensors, and may apply different methods to determine the position for these different axes.

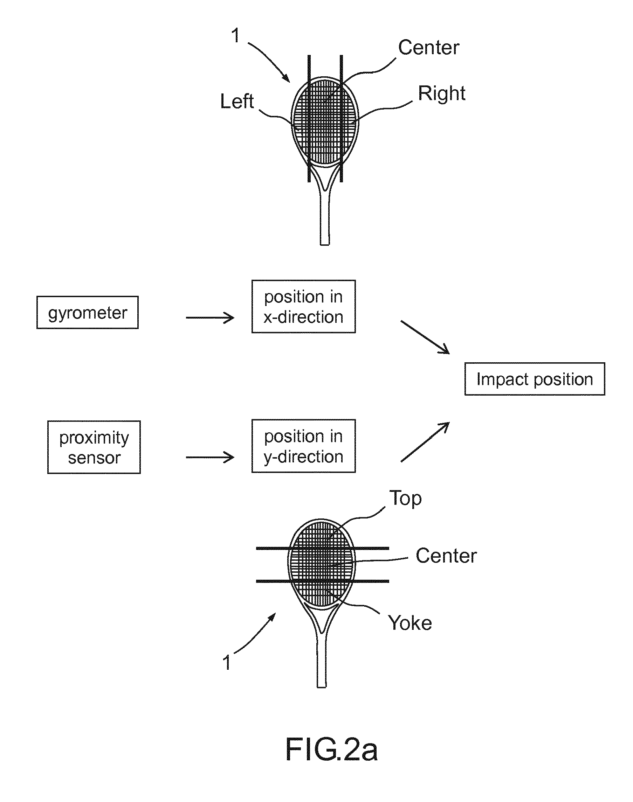

As represented in the bottom part of figure 2a, the proximity sensor is used to determine a position of a ball impact on the racket string bed along the longitudinal Y axis of the racket. For example, the position may be defined as, a top position, a center position, and a yoke or bottom position.

An angular velocity sensor, such as e.g. a gyrometer, may be included in the proximity sensor unit to determine a position of a ball impact on the racket string bed along an axis of the racket, for example the transversal X axis. For example, the position may be defined as, a right position, a center position, and a left position (as represented in the top part of figure 2a). The gyrometer may have three axes, aligned with the axes of the racket in order to measure the rotations (angular velocities) along all axes of the racket.

For example, US 2015/0120021 details, particularly in paragraphs [0154] to [0158], how to determine the impact position in one of several longitudinal impact strips, in this case three longitudinal impact strips along the transversal X axis (top of Figure 2a).

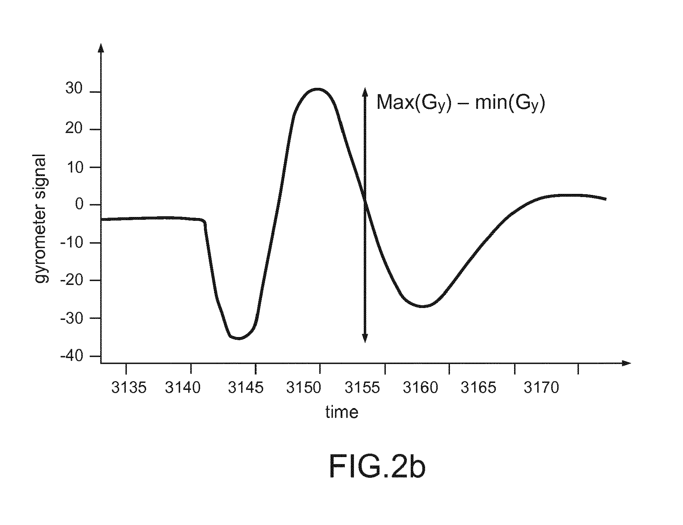

In the event of an impact outside the central strip corresponding to the Y axis, the gyrometer on the Y axis that measures the angular rotational velocity $G_Y$ about the Y axis, displays an oscillation. As represented on figure 2b, a downward slope represents the rotation of the racket caused by the impact of the ball, and the re-ascending slope is due to the overcompensation by the action of the player's wrist.

The amplitude of the oscillation $max(G_Y)$-$min(G_Y)$ is taken as a measurement of the rotation effect due to an impact outside the central axis region. The problem with this measurement is that a high impact velocity slightly outside the axes, and a low-velocity impact near the edge of the racket, have the same effect. This means that it is necessary to compensate for the velocity, i.e. the power of the stroke $E_{GXZ}$ just before impact. This energy may be represented by the following

relationship:

$$E_{GXZ} = |G_X| + |G_Z|$$

wherein

$G_X$ and $G_Z$ represent the angular rotational velocities of rotation about the Xx-and Z-axes. A normalization using $\sqrt{E_{GXZ}}$ works well. To determine if an impact is inside the middle strip or further outside the axis, we introduce C defined by the following relationship:

$$C = \frac{\max(G_Y) - \min(G_Y)}{\sqrt{E_{GXZ}}(t_0 - 0.1s)}$$

A threshold C for example equal to 2 can be used to tell the difference between impacts in the medium band (C < 2), and impacts outside the axes (C > 2). The threshold can be kept fixed, or can be slightly modified (between 1.7 and 2.1) according to the type of stroke (service, backhand, forehand stroke etc.) in order to increase precision. The best threshold is determined by training on a test basis.

By observing the sign of the drift $dG_Y/dt$ at the start of the oscillation, we can determine on which side of the center the impact has taken place.

The method of the invention estimates a position of a ball impact on a racket string bed, along the longitudinal axis of the racket, from data provided by a proximity sensor unit. The unit determines the position of the ball (longitudinal and transversal).

In an embodiment, the data are provided by an accelerometer that is used as a proximity sensor, Indeed an accelerometer can be considered as a proximity sensor because based on the vibrations the distance between the impact position and the position of the sensor may be determined.

The position of a ball impact on the racket string bed, along the longitudinal Y axis of the racket, is estimated from a frequency peak of the acceleration data that is provided.

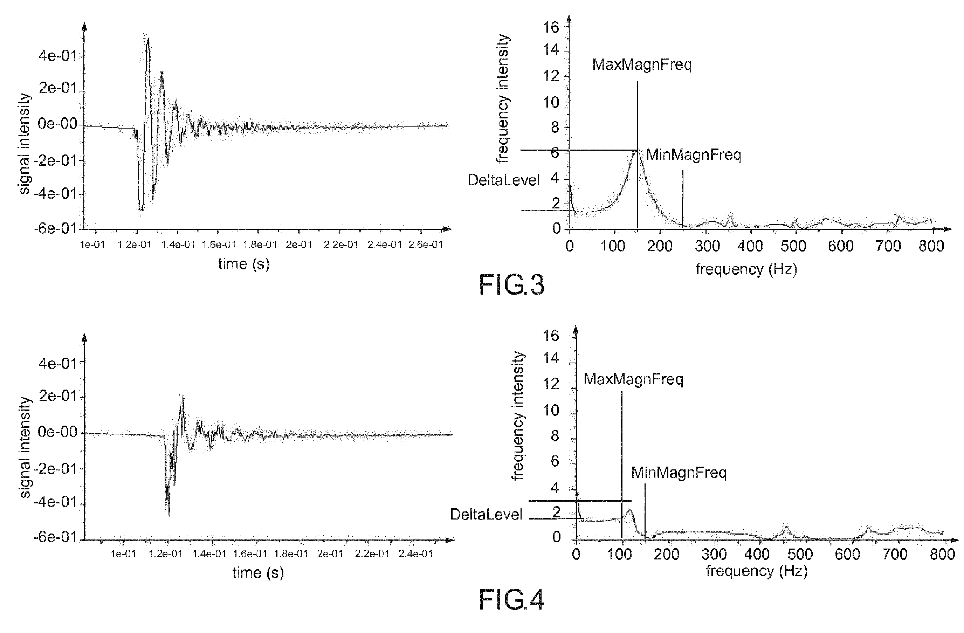

The analysis is, for instance, effected on the Fast Fourier Transform (FFT) between 25Hz and 250Hz. Figures 3 to 6 show examples of the accelerometer signals (left curves) and the FTT (right curves) for different impact positions of the ball on the string bed.

In an embodiment, in the FFT the first maximum and the first minimum in the frequency curves are determined for a frequency between 25Hz and 250 Hz. The magnitudes for the first maximum and for the first minimum are determined. The impact position may be derived from the frequency position in the FFT and the FFT magnitudes of the maximum and the minimum.

In this embodiment, if the frequency of the first minimum in the FFT is greater than the frequency of the first maximum in the FFT, then the position of the ball impact is estimated more at the top of the racket string bed, else the position of the ball impact is estimated more at the yoke of the racket string bed. This means that the frequency position of the minimum with respect to the maximum can be used to determine the impact position in the longitudinal direction. Using this comparison, it can be stated if the impact of the ball was at the top part or the yoke (bottom) part of the string bed.

In addition to the frequency of the minimum and maximum peaks, the magnitude of the peaks may be used to further refine the calculation of the impact position. The magnitude of the minimum and maximum may be compared to each other, or they may be compared to a magnitude at a fixed frequency. Different methods may be used for the top part or the yoke part of the string bed.

For example, when the position of the ball impact is estimated more at the top of the racket string bed, based on the comparison of the frequencies of the minimum and maximum, the magnitude of the first maximum magnitude may be compared to the magnitude at a fixed frequency, in this example 25 hertz. If this difference in magnitude is greater than a first threshold, then the position of the ball impact is estimated at the top of the racket string bed (Figure 3), else the position of the ball impact is estimated at the center of the racket string bed (Figure 4). The difference is indicated as 'Delta Level' in the figures.

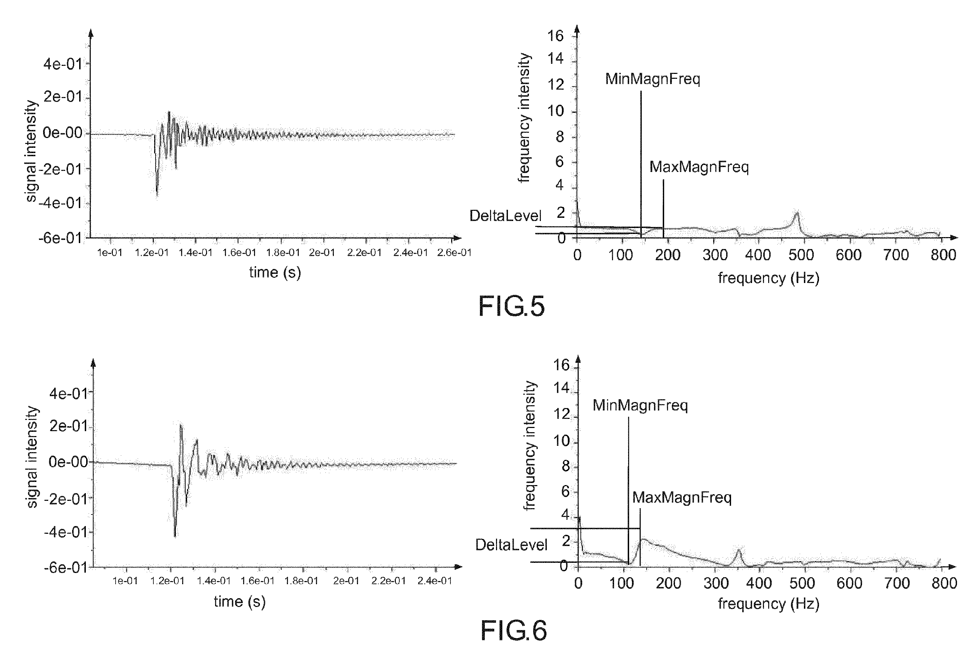

In another example, when the position of the ball impact is estimated more at the yoke of the racket string bed, based on the comparison of the frequencies of the minimum and maximum (or deltalevel), the magnitude of first maximum may be compared to the magnitude of the first minimum. If this difference in magnitude is greater than a second threshold, then the position of the ball impact is estimated at the yoke of the racket string bed (Figure 6), else the position of the ball impact is estimated at the center of the racket string bed (Figure 5).

The exact method applied, and the values of the first and second thresholds may depend on the position of the add-on on the racket module, for example among the four positions Pos1, Pos2, Pos3, and Pos4. The values for the thresholds may also be adapted for the racket and/or the user.

Figure 3 represents the case wherein the ball impact is estimated at the top position of the racket string bed.

Figure 4 represents the case wherein the ball impact is estimated at the center position of the racket string bed using the case of the first threshold.

Figure 5 represents the case wherein the ball impact is estimated at the center position of the racket string bed using the case of the second threshold.

Figure 6 represents the case wherein the ball impact is estimated at the yoke position of the racket string bed.

In these examples, three possible positions can be determined (top, center, and yoke). The amount of possible positions may be increased by applying more thresholds. The limit of the possible positions depends on the signal quality and may depend on the mechanical and/or vibrational characteristics of the racket.

In an alternative embodiment, the positions may be based on an analysis of the shape of the frequency spectrum and a comparison to reference shapes from different known positions. The shape may also be analyzed using curve fitting techniques, and the parameters of the fitting may be compared to parameters of known positions to determine the impact position.

In another embodiment, the longitudinal position is determined using an optical sensor in the proximity sensor unit, comprising at least one light source, such as e.g. a Light Emitting Diode ( LED), and at least one light detector. In this case, the proximity sensor unit may still incorporate an accelerometer in order to determine the orientation of the racket or to determine the impact of a ball by detecting the shock related to the impact. The accelerometer may have three axes, aligned with the axes of the racket.

The position of the ball impact along the longitudinal axis of the racket may be determined from the time of flight of the signals of the optical sensor, or from analyzing the amplitude of the signals of the optical sensor.

The light emitted by a light source is reflected against the tennis ball and detected by the detector. The calculation of the distance can be determined by measuring the amount of reflected light, or by using Time Of Flight (TOF) techniques.

The time of flight method or TOF method may directly yield a distance measurement. The reflection method has to be calibrated using the reflectivity of the tennis ball, and may be different for different balls, or might vary if the reflectivity changes due to wear of the ball. The TOF method is insensitive to the reflection characteristics of the ball.

For the TOF method, the light is emitted in pulses, and the time of flight of the individual pulses is determined. For the reflection method, the light may be emitted continuously or in pulses.

The angle of the light beam determines which part to the racket surface is covered. A wide beam covers a large surface, but the ball may only reflect part of the beam. A narrow beam covers a reduced part of the string bed, but more of the light may be reflected. The proximity sensor unit may produce one large beam from one light source, or several narrow beams from different light sources, to cover the string bed. These different beams may be completely separated or have some degree of overlap.

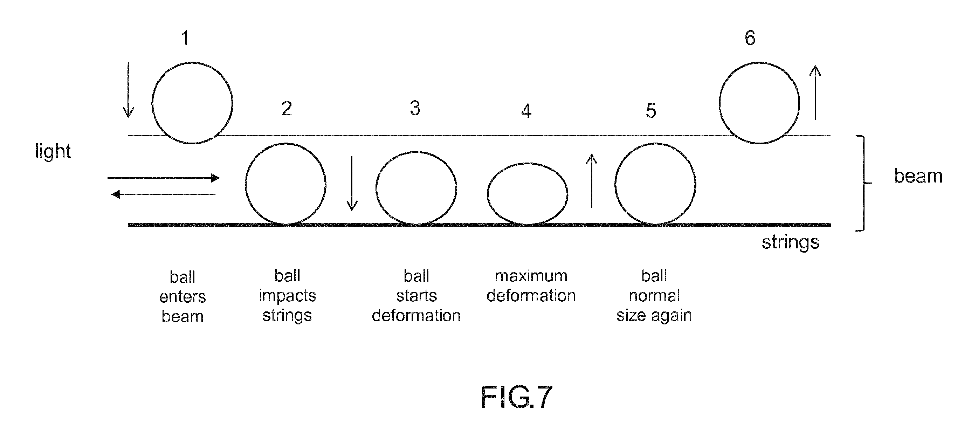

Figure 7 shows the effect of the impact and the bounce of the ball within the light beam.

In position 1, the ball approaches the racquet string bed, and begins to enter in the light beam emitted by the light source. Then the ball crosses the light beam until touching the strings, or in other words the ball impacts the string bed, as illustrated on position 2. Next, the ball starts deforming as illustrated on position 3 until the maximum deformation of the ball as illustrated on position 4. Then the ball returns to its normal size, as illustrated on position 5, and after the rebound, crosses the light beam in the opposite sense until quitting the light beam as illustrated on position 6.

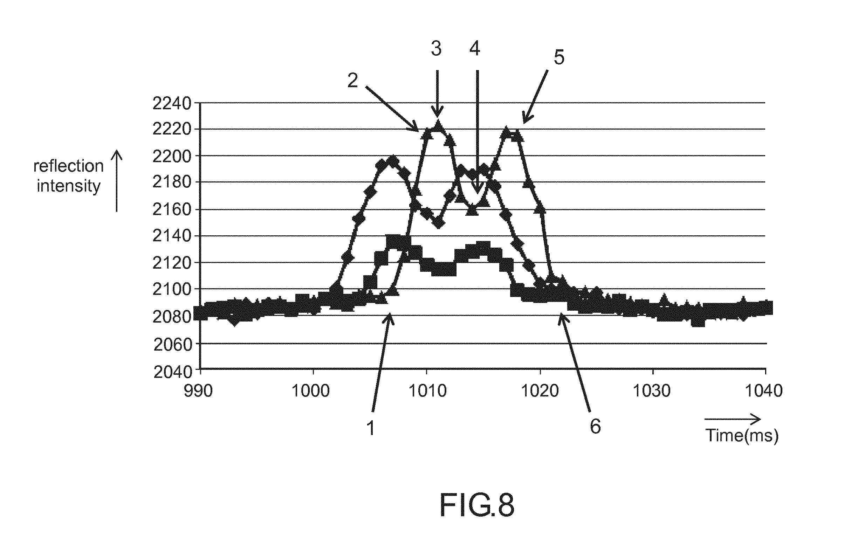

Figure 8 represents several curves corresponding to a ball impact or rebound as illustrated on figure 7. On the highest curve of figure 8 (triangles) are represented the six positions corresponding to the positions described in figure 7.

In the method of analyzing the amplitude of the signals of the optical sensor, the amplitude of the reflection signal is a measure of the distance. The typical signal curves show a local minimum, or saddle, because when a ball impacts the racket, the ball deforms and its reflection surface decreases.

The decreased surface results in a decrease of the amount of reflected light, and thus creates a local minimum when the deformation of the ball is at its maximum. The local minimum reflects the point in time where the ball changes direction, from moving towards the string bed to moving away from the string bed.

To compensate for the ambient light, measurements may be taken with the light source(s) off, and the detected light levels may then be subtracted from the actual measurements with the light source(s) on. These corrections for the ambient light may be done at a predetermined time interval. Alternatively, the ambient light correction may e.g. be done at the start of the swing, which may be determined using the motion sensors. The ambient light correction may also be done before or after the impact of the ball, in order to measure the ambient light as close as possible (in orientation) to the actual measurement. In this case, the ambient light measurements may be triggered by the ball impact, as detected e.g. by the motion sensors.

Measurements with the light source(s) on, but without the ball, may be taken to correct for the reflection of any parts of the racket itself, such as the racket frame or the string bed.

The proximity sensor unit may have a single light source that can cover the impact of the ball on both sides of the string bed. In some embodiments, a light source may be required on both sides of the string bed in case the strings would be an obstruction for the light when the ball is on the opposite side of the strings compared to the light source.

Once the position of the ball impact in the longitudinal direction Y has been derived by the proximity sensor unit, and the position in the transversal direction x has been determined using the gyrometers, the locations in both directions x and y can be combined to determine the impact position of the ball on the racket along the two directions (as indicated in Figure 2a).

A calibration can be effected to match the amplitude of the reflection and the corresponding position of a ball impact on the racket string bed, along the longitudinal axis Y of the racket 1. This calibration may be done by placing a ball at different positions on the string bed and measuring the reflected signal. These calibration measurements may be done using different balls, for example with different degrees of wear, to take this influence into account. The calibration distance results may be expressed in actual distance units, or in relative units with respect to the racket frame or string bed dimensions. The calibrations may be done at the factory by the manufacturer and/or by the user. The user may be prompted to perform one or more calibration manipulation, for example every N times the user powers on the device, or at a certain time interval.

The calibration may also be an automated process. For example, the system may record the impacts and determine the relative impact position from the distribution of intensities. The recorded impact with the lowest intensity is linked to the impact position closest to the yoke (assuming the proximity sensor unit is positioned at the yoke or further down on the handle). The recorded impact with the highest intensity is linked to the impact position closest to the top of the racket. When starting to play, the lowest and highest intensity may not correspond to the actual bottom and top of the racket, but assuming that during normal play the impact position will cover (more or less) the complete string bed, at the end of play the lowest and highest intensity may approximately correspond to the bottom and top of the racket. This means that during play the lowest and highest recorded intensity may change, and the relative position of the impact may have to be recalculated.

The light source(s) in the proximity sensor unit may require a lot more power compared to the rest of the sensors. For example, the consumption of a LED is in the order of several tens of mA (e.g. 50mA), while a gyrometer consumes only a few mA (e.g. 3mA). Therefore, measures may be taken to reduce the power consumption. For example, the light source may only be on during a tennis swing. In this case, the motion sensor will trigger the light source(s) when a swing is detected.

For example, it is possible to measure the acceleration or angular velocity, and activate the light source(s) above a preset threshold. Alternatively, the motion sensor may perform some sort of gesture recognition, and when a tennis swing is detected the light source(s) are triggered. Additionally, if the proximity sensor unit has light sources on both sides of the string bed, it is possible to only use the light source on the side of the racket where the impact is going to be. This side may be determined using the motion sensor in the racket. For example, the gyrometer on the x-axis measures the direction of the angular rotation. A positive angular velocity may mean that the user moves the racket in one direction, and a negative angular velocity may mean that the user moves the racket in the other direction. Thus, the sign of the angular velocity may be used to determine which light source to operate. When the user makes a swing, he or she may move the racket back before making the swing. This backward motion is in general slower than the forward motion to hit the ball. Therefore, a threshold may be used only to operate the light source during the faster forward motion when the user has the intention to hit the ball.

In an alternative embodiment, a light source can be put on only when an impact is detected, for example using an accelerometer and e.g. detecting an acceleration above a preset threshold value. This means that the light source(s) would be activated at approximately the first maximum of the reflected signal because this maximum is caused by the decrease in reflection caused by the deformation of the ball during the impact (position 2 and 3 in Figures 7 and 8). This is sufficient to determine the impact position using the signal intensity because the second maximum may be used as a measure for the reflected intensity (position 5 in Figures 7 and 8).

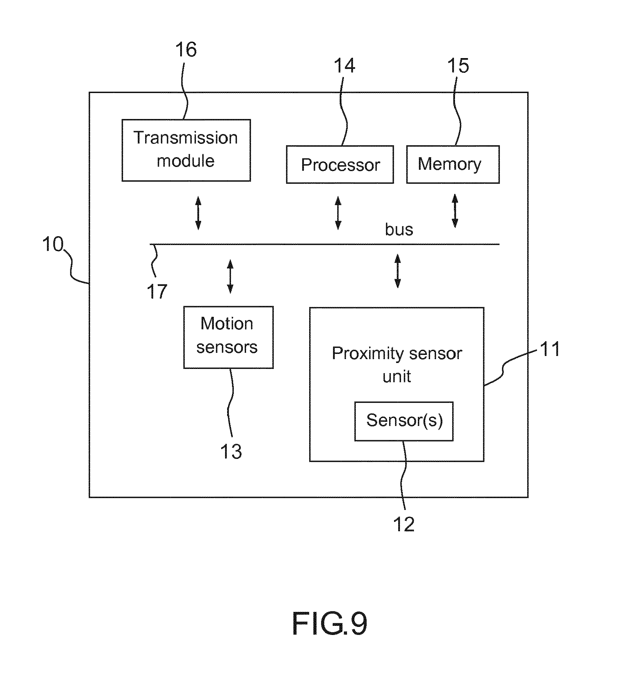

The impact position on the string bed may be determined as a function of the type of tennis swing, such as e.g. serve, forehand, backhand, etc. The type of swing may be determined using the motion sensor in the proximity sensor unit. For example, the type of swing may be determined using the accelerometer and the gyrometer data. This is explained in detail in patent application US 2015/0120021 A1 in the discussion of figures 8 and 9 of the application and paragraphs [0141] to [145].

To tell a forehand stroke apart from a backhand, three factors are preferably taken into account:

> first of all, the dominant hand: it is desirable to determine whether the player is left- or right-handed. It is desirable to tell between a forehand stroke of a right-handed player and a backhand stroke of a left-handed player, as well as between a backhand stroke of a right-handed player and a forehand stroke of a left-handed player. The dominant hand can be deduced from the swing of the service, or can be known from an input by the user;
> next the orientation of the racket 1: to determine the orientation of the racket, the orientation of the X axis of the racket with respect to the gravity vector G is computed. The sign of the projection of the X axis onto G gives us the orientation of the racket 1 as illustrated in Figure 8 of application US 2015/0120021 A1, the x-axis is vertical oriented downward when the projection of the X axis onto G has a value of 1 and the x-axis is vertical oriented upward when the projection of the X axis onto G has a value of -1;
> finally, the direction of the movement before the impact: the direction of the movement is determined from the sign of the angular rotational velocity supplied by the gyrometer G relative to the X axis.

If the value 1 is associated with a right-handed player and the value -1 with a left-handed player, it is possible to determine the type of stroke by multiplying these three factors. If the result is positive (i.e. has a value of 1) the stroke is a forehand stroke, and if the result is negative (i.e. has a value of -1) the stroke is a backhand stroke.

Both types of sensors used in the proximity sensor unit, the accelerometer or the optical sensor, can be used to determine the impact position. However, the proximity sensor may in addition be used to determine the ball speed before and after the impact. The ball speed is determined by measuring the time it takes the ball to traverse the light beam. The ball enters the light beam when the reflected signal intensity starts to increase, as shown at position 1 in Figures 7 and 8. The ball impacts the string-bed at position 2, which means that the derivation of the reflected signal intensity starts to decrease. (It is assumed here that the ball is larger than the beam size, otherwise a plateau would be seen around position 3). Position 2, i.e. the ball impact, may also be deduced from the motion sensors. In addition, the width of the light beam that the ball traverses needs to be known or estimated. This means that by determining the time interval between position 1 and 2 (or 3), the incoming speed of the ball can be determined. In a similar manner, the outgoing speed of the ball can be determined, using the time interval between the positions 5 and 6 in the figures. By comparing the incoming and outgoing speeds, the energy win or loss can also be determined. If the width of the beam is not known, only the relative gain or loss of speed of the ball may be determined. This ball speed information may also be combined with the speed of the racket determined by the motion sensors.

In the TOF method, the signal form is different, but the ball incoming and outgoing speeds may still be determined. The first measurements would be of the bottom of the ball as it enters the detection beam. As the ball moves towards the racket and further into the ball, the center of the ball is determined, which would yield a smaller distance. Then, when the ball deforms, the diameter parallel to the racket increases, and the distance decreases to a minimum. Next, the process is reversed. These features in the signal may be used to calculate the ball speed as in the reflective method. The time of impact as measured by the motion sensors may also be used to determine the time interval with the moments the ball enters and leaves the beam in order to calculate the incoming and outgoing speed.

Figure 9 shows an example architecture of the add-on device. The device 10 contains the proximity sensor unit 11 which incorporates the required sensor(s) 12, which may be for instance the accelerometer or optical sensors as described above. Motion sensors 13, such as a gyrometer or an accelerometer, are depicted outside the proximity sensor unit 11, but may instead by contained in the unit. The various calculation may be done in the add-on device, or externally. The add-on device may include a processor 14 that performs all the required calculations and calibration. The algorithms may be stored in a memory 15, which may also be used to store the measured data, in a raw format or a processed format. The impact position and the types of tennis swing, ball speeds etc. may all be stored in the memory. A transmission module 16 is also provided to transfer the data to another device which may analyze the data further and present the results to the user/player. The data transmission may be wired or wireless, using any suitable protocol (e.g. Bluetooth). The different components of the add-on device may communicate over a bus 17. Although most of the components are shown external to the proximity sensor unit, all, or some of them, may be incorporated in the unit.

While this invention has been particularly shown and described with references to example embodiments thereof, it will be understood by those skilled in the art that various changes in form and details may be made therein without departing from the scope of the invention encompassed by the appended claims. For example, the invention may be applied to other sports equipment where the impact position of an object may be determined.

**Claims**

1. Method for estimating a position of a ball impact on a racket string bed, along the longitudinal axis (Y) of the racket (1), from data provided by a proximity sensor unit.

2. Method according to claim 1, wherein said data are provided by an accelerometer.

3. Method according to claim 2, wherein said position of a ball impact on the racket string bed, along the longitudinal axis (Y) of the racket (1), is estimated using a frequency analysis of data provided by the accelerometer.

4. Method according to claim 3, wherein, if the frequency of the first minimum magnitude in the FFT curve corresponding to the provided data is greater than the frequency of the first maximum magnitude of the FFT curve corresponding to the provided data, then the position of the ball impact is estimated more at the top of the racket string bed, else the position of the ball impact is estimated more at the yoke of the racket string bed.

5. Method according to claim 4, wherein, if the position of the ball impact is estimated more at the top of the racket string bed and if the difference between said first maximum magnitude and the magnitude at a fixed frequency is greater than a first threshold, then the position of the ball impact is estimated at the top of the racket string bed, else the position of the ball impact is estimated at the center of the racket string bed.

6. Method according to claim 4 or 5, wherein if the position of the ball impact is estimated more at the yoke of the racket string bed, if the difference between said first maximum magnitude and said first minimum magnitude is greater than a second threshold, then the position of the ball impact is estimated at the yoke of the racket string bed, else the position of the ball impact is estimated at the center of the racket string bed.

7. Method according to claim 1, wherein said data are provided by an optical sensor comprising at least one light source and at least one light detector.

8. Method according to claim 7, wherein said position along the longitudinal axis (Y) of the racket is determined from the time of flight of the signals of the optical sensor.

9. Method according to claim 7, wherein said position along the longitudinal axis (Y) of the racket is determined from analyzing the amplitude of the signals of the optical sensor.

10. Method according to anyone of claims 7 to 9, wherein the light source is controlled based on data provided by at least one of a gyrometer and an accelerometer.

11. Method according to anyone of claims 7 to 10, wherein the light source is on, during an interval around a ball impact on a racket string bed.

12. Method according to one of claims 7 to 11, wherein at least one of an incoming ball speed and an outgoing ball speed is determined based on said data.

13. Method according to anyone of claims 1 to 12, comprising an estimation of a transversal position of a ball impact along a transversal axis (X).

14. System for estimating a position of a ball impact on a racket string bed, comprising :

   - an add-on module adapted to be mounted on a racket, the add-on module comprises a proximity sensor unit;
   - a calculator adapted to estimate the position of a ball impact on the racket string bed, from data provided by the proximity sensor unit.

15. System according to claim 14, wherein the proximity sensor comprises an accelerometer.

16. System according to claim 14, wherein the proximity sensor comprises at least one light source and at least one light detector.

FIG.1

1

Center

Left

Right

gyrometer → position in x-direction

proximity sensor → position in y-direction

Impact position

Top

Center

Yoke

1

# FIG.2a

FIG.2b

FIG.3

FIG.4

FIG.5

FIG.6

EP 3 153 981 A1

FIG.7

FIG.8

FIG.9

Europäisches
Patentamt

European
Patent Office

Office européen
des brevets

# EUROPEAN SEARCH REPORT

Application Number

EP 15 30 6560

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/251054 A1 (SCHNEIDER REINHARD [AT] ET AL) 10 September 2015 (2015-09-10) * paragraph [0021] - paragraph [0026] * * figures 1A,2,3 * | 1-6, 13-15 | INV. G06F19/00 |
| X | US 2011/021280 A1 (BORODA VLADIMIR [IL] ET AL) 27 January 2011 (2011-01-27) | 7-10,12, 16 | |
| Y | * paragraphs [0011], [0047], [0050], [0069], [0070]; figure 4 * | 11 | |
| X | US 2007/105664 A1 (SCHEINERT STEFAN [US] ET AL) 10 May 2007 (2007-05-10) | 7 | |
| Y | * paragraph [0032]; claim 6 * | 11 | |
| X | US 4 101 132 A (CONREY RICHARD N ET AL) 18 July 1978 (1978-07-18) * column 5 - lines 5-20 * * column 6, paragraph 3 * * column 9, paragraph 3; figure 12 * | 7,8,16 | |

TECHNICAL FIELDS SEARCHED (IPC)

G06F

The present search report has been drawn up for all claims

| Place of search | Date of completion of the search | Examiner |
|---|---|---|
| Munich | 17 August 2016 | Laub, Christoph |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................
& : member of the same patent family, corresponding
document

EPO FORM 1503 03.82 (P04C01)

Europäisches
Patentamt

European
Patent Office

Office européen
des brevets

Application Number

EP 15 30 6560

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

**Europäisches Patentamt**
**European Patent Office**
**Office européen des brevets**

**LACK OF UNITY OF INVENTION**
**SHEET B**

Application Number

EP 15 30 6560

The Search Division considers that the present European patent application does not comply with the requirements of unity of invention and relates to several inventions or groups of inventions, namely:

1. claims: 1-6, 13-15

    Determination of the impact point of a ball against a racket from mechanical information
    ---

2. claims: 7-12, 16

    Determination of the impact point of a ball against a racket from non-mechanical information
    ---

## ANNEX TO THE EUROPEAN SEARCH REPORT
## ON EUROPEAN PATENT APPLICATION NO.

EP 15 30 6560

This annex lists the patent family members relating to the patent documents cited in the above-mentioned European search report.
The members are as contained in the European Patent Office EDP file on
The European Patent Office is in no way liable for these particulars which are merely given for the purpose of information.

17-08-2016

| Patent document cited in search report | | Publication date | Patent family member(s) | | Publication date |
|---|---|---|---|---|---|
| US 2015251054 | A1 | 10-09-2015 | DE 102014003353 B3 | | 10-09-2015 |
| | | | EP 2916100 A1 | | 09-09-2015 |
| | | | US 2015251054 A1 | | 10-09-2015 |
| US 2011021280 | A1 | 27-01-2011 | NONE | | |
| US 2007105664 | A1 | 10-05-2007 | NONE | | |
| US 4101132 | A | 18-07-1978 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150157901 A1
- US 20150120021 A

- US 20150120021 A1